# EUROPEAN PATENT APPLICATION

(11) **EP 0 820 747 A1**
(43) Date of publication of application: **28.01.1998**
(21) Application number: 96111969.0
(22) Date of filing: 25.07.1996
(51) Int. Cl.: A61F 13/15

(54) **Absorbent article with elastic feature**

(71) Applicant: THE PROCTER & GAMBLE COMPANY, Cincinnati, Ohio 45202 (US)
(72) Inventor: Müller, Jörg, 61184 Karben (DE); Soon, See-Aun, 65818 Schwalbach (DE)
(74) Representative: Canonici, Jean-Jacques

(57) **Abstract**

An absorbent article having an improved elastic feature which is easy to handle during manufacture and subsequent processing of the absorbent article. The composite elastic feature comprises at least one extruded elastomeric layer and at least one extruded, substantially non-elastomeric layer which form a film. Before the film is elasticized by activation the non-elastomeric layer inhibits the elastic properties of the elastomeric layer, said inhibiting effect being lost after activation. The absorbent article can be any absorbent article having an elastic feature, preferably integral disposable absorbent articles.

## Description

### FIELD OF THE INVENTION

The present invention relates to absorbent articles such as diapers, incontinent briefs, training pants, and the like, and more particularly to absorbent articles having at least one elastic feature which comprises an extruded film of at least one elastomeric layer extruded with at least one non-elastomeric layer. The elastic properties allow for improved control during the manufacture of the absorbent articles.

### BACKGROUND OF THE INVENTION

The major function of absorbent articles such as disposable diapers and adult incontinent briefs is to absorb and contain body exudates. Such articles are thus intended to prevent body exudates from soiling, wetting, or otherwise contaminating clothing or other articles, such as bedding, that come in contact with the wearer. Absorbent articles thus function both to contain the discharged materials and to isolate these materials from the body of the wearer and from the wearer's garments and bed clothing. However, absorbent articles have a tendency to sag or gap away from and to slide/slip down on the body of the wearer during wear. This sagging/gapping and sliding/slipping is caused by the relative motions of the wearer as the wearer breathes, moves and changes positions, by the downward forces generated when the absorbent article is loaded with body exudates, and by the deformation of the materials of the absorbent article itself when subjected to such wearer's motions. This sagging/gapping and sliding/slipping of the absorbent article can lead to premature leakage and poor fit of the absorbent article about the wearer in the waist regions and the leg regions of the absorbent article.

In order to more snugly fit absorbent articles about the wearer, certain commercially available absorbent articles have been provided with elastic features. An example of a disposable diaper with an elastomeric waist feature which has achieved wide acceptance and commercial success is disclosed in U.S. Patent 4,515,595 issued to Kievit and Osterhage on May 7, 1985. Elastic waist features will typically comprise an elasticized waistband consisting of an elastomeric member contractibly affixed between the topsheet and the backsheet. The elasticized waistband is, thus, designed to expand and contract with the wearer's motions and to maintain the fit of the absorbent article about the waist of the wearer during use. Disposable absorbent articles having elastic leg features are also known to the art. For example, U.S.A. Patent 3,860,003, entitled "Contractable Side Portions For Disposable Diaper", issued to Buell on January 14, 1975, describes an absorbent article having an elasticized leg cuff which has achieved wide acceptance and commercial success.

Methods for stretching an elastic feature are described in U.S. Patent 4,107,364, issued to Sisson on August 15, 1978, U.S. Patent 4,209,563 issued to Sisson on June 24, 1980, U.S. Patent 4,525,407 issued to Ness on June 25, 1985, U.S. Patent 4,834,741 issued to Sabee on May 30, 1989, European Patent Publication 409,315 in the name of the Procter & Gamble Company, published January 23, 1991; all are hereby incorporated by reference.

Still other methods are described in U.S. Patent 5,167,897, U.S. Patent 5,156,793, and United States Serial no. 07/662543, each filed February 28, 1991 and assigned to The Procter & Gamble Company, which are also incorporated herein by reference. These patents describe making an elasticized laminate by mechanically stretching a zero-strain stretch laminate web to impart the elasticity thereto in the direction of stretching, at least up to the point of initial stretching. An elongatable, non-elastic nonwoven layer is first bonded to a liquid-impervious elastic layer while in its relaxed state to form the laminate web. The laminate web is then fed between a pair of opposed pressure applicators, preferably ones having three-dimensional surfaces which are complimentary to a varying degree with one another, and subjected to incremental mechanical stretching (or nonuniform or sequential mechanical stretching, as desired), whereby the elongatable nonwoven is permanently elongated in the direction of stretching. When the mechanical stretching is removed, the elastic layer will enable the laminate to return substantially to its pre-stretch shape and dimensions, thereby rendering the non-elasticized laminate elastically extensible in the direction of initial stretching.

Conventionally, the elastic feature of an absorbent article consists of a laminate web of at least one elongatable, non-elastic nonwoven layer and at least one elastic layer with both layers in their relaxed state. The laminate web is elasticized by mechanically stretching at least portions of the laminate web along its width to impart elasticity thereto in the direction of stretching, at least up to the point of initial stretching. The elastomeric web may first undergo a "cut and slip" operation prior to activation by mechanical stretching, so that it has the required dimensions as the elastic feature of the absorbent circle. Such operations are described in U.S. 5,167,897 and U.S. 5,156,793 and additionally in U.S. 5,143,679.

A multi-layer elastomeric laminate is described also in U.S. 5,462,708. The laminate comprises at least one elastomeric core layer and at least one relatively non-elastomeric skin layer. The skin layer is stretched beyond its elastic limit and recovered in selected non-deactivated areas with the core layer so as to form elastic regions. The microtexture structure of the laminate of U.S. 5,462,708 means that the layer contains peak and valley irregularities or folds.

Although such conventionally used elastomeric laminates provide satisfactory elastic and tactile properties, a number of disadvantages are associated with their use.

The elastomeric laminate first has to be prepared before entering the steps leading to the manufacture of an absorbent article. Conventionally, this is carried out by adhering the nonwoven layer to the elastic layer. The laminate is then assembled to form the absorbent article, typically by glueing the laminate to at least a part of the backsheet and/or top sheet of the article. Adhesive bonds occur between the fibres of the nonwoven layer, leading to weak points in the structure which are susceptible to tearing when a sufficient amount of tension is applied to the absorbent article.

Furthermore, the tensile strength of the nonwoven layer can be relatively weak, thus making it difficult to control the elastomeric properties of the elastomeric layer, for example, during a "cut and slip" operation, during assembly of the absorbent article and/or during mechanical stretching of the elastomeric laminate.

Additionally, during the manufacture of the elastomeric laminate, the use of a non-woven layer and the use of glue to combine the non-woven layer and elastomeric layer can lead to contamination of the machinery both from the fibres and from the glue.

It is, therefore, an object of the present invention to provide an absorbent article having an elastic feature with improved properties. Furthermore, it is an object of the invention to provide an absorbent article which has an elastic feature which is easier to handle and to process. It is a further object to provide an absorbent article in which the layers of the elastic feature are more easily joined together, particularly adhered together, and in addition, are more easily joined to other features of the absorbent article, particularly to the topsheet or backsheet, due to an increased bond strength, resulting in an absorbent article having improved tensile strength, reduced tearability, but still having excellent containment characteristics and acceptable tactile properties to the wearer. The invention additionally aims to provide an absorbent article which has an elastic feature which has both improved and more consistent elastic properties due to the improved control of the elastomeric material during processing and easier activation to result in improved stretch properties. Hence, the invention aims to provide an elastic feature having properties resulting in easier processing, combined with more cost effectiveness, due to the avoidance, in particular, of the use of the non-woven layer conventionally used.

### SUMMARY OF THE INVENTION

The absorbent articles of the invention are preferably integral disposable absorbent articles which are articles which absorb and contain body exudates and more specifically refer to articles which are placed against or in proximity to the body of the wearer to absorb and contain the various exudates discharged from the body and which are intended to be discarded after a single use (i.e., they are not intended to be laundered or otherwise restored or reused), and which are unitary in that they do not require separate manipulative parts like a separate holder and liner. The invention additionally can be extended to any hygienic article, which is disposable or at least partially reusable. The invention can also be extended to cover multipiece products.

The present invention provides absorbent articles such as disposable diapers, incontinent briefs, sanitary napkins and diaper holders, having a unique elastic feature. Such absorbent articles comprise a containment assembly which comprises a liquid pervious topsheet, a liquid impervious backsheet joined with said topsheet, an absorbent core having side edge and waist edges and being positioned between the topsheet and the backsheet together with a composite elastic feature which comprises at least one elastomeric layer and at least one layer which is substantially non-elastomeric, wherein said layers are extruded to form a film, preferably said layers are co-extruded to form a film, and wherein said film is elasticized by activation, so that the substantially non-elastomeric layer, which when combined with the elastomeric layer has a restraining or inhibiting effect on the elastic properties thereof, loses or partially loses this restraining effect so that the composite elastic feature can be stretched beyond its initial dimensions existing prior to activation and additionally can retract due to the contractive forces of the elastomeric layer. The elastic feature preferably comprises a non-elastomeric layer which is sufficiently brittle that on activation, regions of the non-elastomeric layer break to free the elastic properties of the elastomeric layer of the film. Hence, it is a preferred feature of the invention that upon activation with any mechanism available, and preferably these methods described herein, the non-elastomeric layer or layers of the composite elastic feature is broken due to its brittle properties at distinct points leading to non-continuous, non-elastomeric film layers in the elastic composite or laminate structure. In its relaxed state, the elastomeric film of the composite retracts so that the break points in the non-elastomeric film are not exposed. On stretching, the non-elastomeric film, which is preferably non-elongatable or at least less elongatable than the elastomeric film, can be stretched due to the break points revealing a non-continuous film. Hence, the properties of the non-elastomeric film are such that prior to activation, the non-elastomeric film restricts the elastic properties of the elastomeric film in the composite. After activation, the break points free the elastic properties. The elastomeric layer and non-elastomeric layer are extruded or co-extruded to form a laminate or composite structure.

It is preferable that the non-elastomeric layer of the composite elastic feature is non-elongatable or at least being less elongatable (regardless of whether this is elastically or plastically elongatable) than the elastomeric layer. Hence, due to the properties of the non-elastomeric layer, before activation of the extruded film, the non-elastomeric layer inhibits the elastic properties of the elastomeric layer. Once the film has been activated, for example, by mechanical activation, the non-elastomeric layer loses its restraining effect on the elastomeric layer freeing the elastic properties.

The non-elastomeric layer, therefore, may be any conventionally available polymer which is non-elongatable or is less elongatable than the elastomeric layer forming the film. For example, for a suitable non-elastomeric material which may be suitable as the non-elastomeric layer in the elastic film, the value of force at break point may preferably be less than 1.0 N/mm² and more preferably less than 0.3 N/mm², and the elongation value at break may preferably be less than 5%, more preferably, less than 2%.

The elastic feature of the absorbent article is preferably situated between a portion of the topsheet and backsheet of the absorbent article, preferably extending outwardly from at least one of the edges of the absorbent core.

According to an embodiment of the invention, the elastic feature may comprise an elasticized leg cuff extending laterally outwardly from one of said side edges of said absorbent core, an elastic waist feature extending longitudinally outwardly from at least one of the waist edges of the absorbent core, and/or elasticized side panels, wherein each of the side panels is elastically extensible in the lateral direction. Optionally, the whole or part of the backsheet of the absorbent article can comprise the composite elastic film of the invention, which may be subsequently activated in selected regions.

Alternatively, the elastic film can be combined or be integral with part or all of the topsheet of the absorbent article and activated in specific regions. In the latter case, the elastic film may comprise apertures so that the topsheet is pervious.

The extruded film may comprise any number of elastomeric and non-elastomeric layers depending on the required elastic properties of a particular absorbent article. For example, the extruded film may be formed from any combination of one non-elastomeric layer with one elastomeric layer, one elastomeric layer with two non-elastomeric layers one on either side of said elastomeric layer or alternatively, one non-elastomeric layer with two elastomeric layers, one on either side of said non-elastomeric layer.

The substantially non-elastomeric layer and elastomeric layer are extruded to form a film. Preferably, the two are combined to form a film in one process step, for example, by co-extrusion, for example, by each of the layers being extruded from the same die head. Alternatively, two or more die heads can be used, with a subsequent step of combining two separately extruded layers to form a film after extrusion, for example, by heat bonding.

The film can be activated by any known method, for example, by mechanical stretching, such as, by stretching in the machine direction, for example, by stretching between two pairs of nip rolls or by stretching in the cross machine direction, for example, by running the film through a tentering frame or preferably, by the technique known as "ring-rolling" which is described, for example, in U.S. 5,156,793, 5,167,897 and 5,143,679, referred to previously and incorporated herein by reference. Alternatively, activation can be carried out by any method which overcomes the restraining force of the substantially non-elastomeric layer on the elastic properties of the elastomeric layer, for example, by weakening due to cutting or scratching the surface of the film, weakening by UV-light "deadening", applying heat, as required, during activation, by activation in use or through action by the user or carer, for example, the mother, etc.

The invention will now be described in more detail with reference to the drawings depicting embodiments of the invention, for illustrative purposes only.

### BRIEF DESCRIPTION OF THE DRAWINGS

Figure 1 is a plan view of a disposable diaper having portions cut away to reveal underlying structure, the outer surface of the diaper facing the viewer;
Figure 2 is a perspective view of a disposable training pant embodiment of the invention in a typical in-use configuration as it would be applied to a wearer;
Figure 3 is a plan view of the chassis of the training part of Figure 2 having portions cut away to reveal the underlying structure, the surface which will form the outer surface of the disposable garment facing away from the viewer;
Figure 4 is a simplified perspective view showing the assembly of a web of single use diapers, each having elastomeric patches secured thereto at regularly spaced locations along its length, said web being subjected to a sequential stretching process using multiple pairs of meshing corrugated rolls in the areas of said web coinciding with the elastomeric patches, said web also being cut at predetermined points along its length to form a multiplicity of single use diapers, each having at least one pair of laterally stretchable side panels;
Figure 5 is a simplified perspective view of a sequential web stretching assembly of the present invention, said assembly being comprised of two sequentially positioned sets of meshing corrugated rolls;
Figure 5A is a simplified view taken along view line 2A-2A in Figure 5 and showing the manner in which idler rolls are used to cause the diaper web to wrap lowermost corrugated rolls;
Figure 5B is a highly enlarged view taken at the inset 2B shown in Figure 5, said view showing the degree of meshing of the first set of corrugated rolls with one another as the "zero-strain" stretch laminate portion of the diaper web passes therebetween;
Figure 5C is a highly enlarged view taken at the inset 4C shown in Figure 5, said view showing the degree of meshing of the second set of corrugated rolls with one another as the partially stretched "zero-strain" stretch laminate portion of the diaper web passes therebetween;
Figure 6 is a cross sectional view of an extruded laminate (a) before activation, (b) after activation, and (c) in its relaxed state after activation.

### DETAILED DESCRIPTION OF THE INVENTION

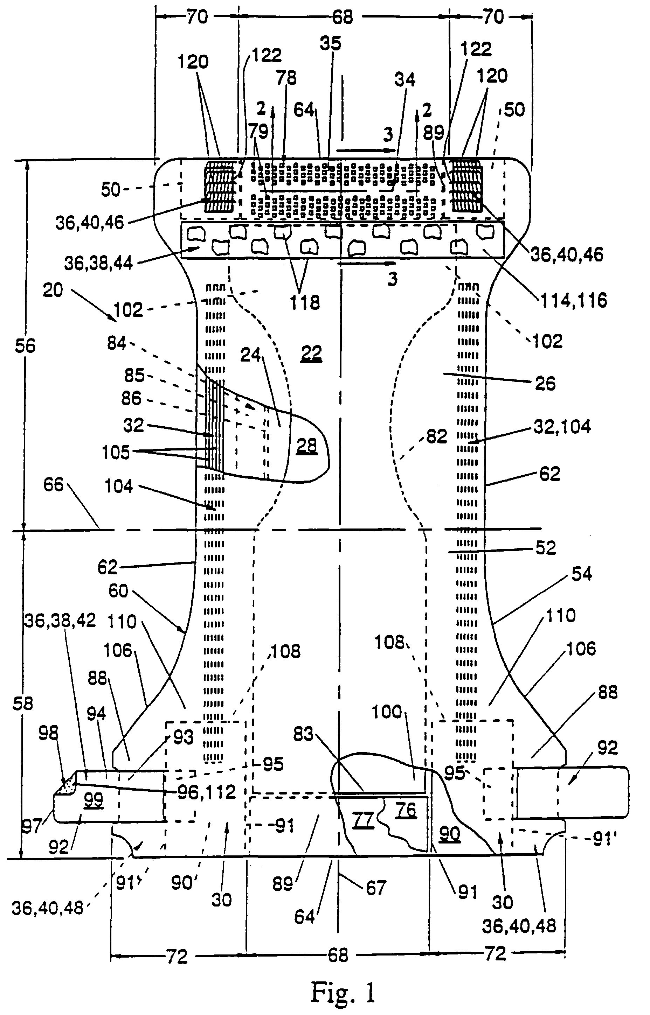

An example of an absorbent article of the present invention is the unitary disposable absorbent article, a diaper, shown in Figure 1. As used, herein, the term "diaper" refers to an absorbent article generally worn by infants and incontinent persons that is worn about the lower torso of the wearer. It should be understood, however, that the present invention is also applicable to other absorbent articles such as incontinent briefs, incontinent undergarments, diaper holders and liners, feminine hygiene garments such as sanitary napkins and pantiliners, and the like.

Figure 1 is a plan view of an embodiment of an absorbent article of the invention which is a diaper.

The diaper 20 is shown in Figure 1 in its flat-out, uncontracted state (i.e. with elastic induced contraction pulled out except in the side panels wherein the elastic is left in its relaxed condition) with portions of the structure being cut-away to more clearly show the construction of the diaper 20 and with the portion of the diaper 20 which faces away from the wearer, the outer surface 52, facing the viewer. As shown in Figure 1, the diaper 20 comprises a containment assembly 22 preferably comprising a liquid pervious topsheet 24, a liquid impervious backsheet 26 joined with the topsheet 24, and an absorbent core 28 positioned between the topsheet 24 and the backsheet 26; elasticized side panels 30; elasticized leg cuffs 32; an elastic waist feature 34; and a closure system comprising a dual tension fastening system generally multiply designated as 36. The dual tension fastening system 36 preferably comprises a primary fastening system 38 and a waist closure system 40. The primary fastening system 38 preferably comprises a pair of securement members 42 and a landing member 44. The waist closure system 40 is shown in Figure 1 to preferably comprise a pair of first attachment components 46 and a second attachment component 48. The diaper 20 also preferably comprises a positioning patch 50 located subjacent each first attachment component 46.

The diaper 20 is shown in Figure 1 to have an outer surface 52 (facing the viewer in Figure 1), an inner surface 54 opposed to the outer surface 52, a first waist region 56, a second waist region 58 opposed to the first waist region 56, and a periphery 60 which is defined by the outer edges of the diaper 20 in which the longitudinal edges are designated 62 and the end edges are designated 64. (While the skilled artisan will recognize that a diaper is usually described in terms of having a pair of waist regions and a crotch region between the waist regions; in this application, for simplicity of terminology, the diaper 20 is described as having only waist regions, each of the waist regions including a portion of the diaper which would typically be designated as part of the crotch region). The inner surface 54 of the diaper 20 comprises that portion of the diaper 20 which is positioned adjacent to the wearer's body during use (i.e. the inner surface 54 generally is formed by at least a portion of the topsheet 24 and other components joined to the topsheet 24). The outer surface 52 comprises that portion of the diaper 20 which is positioned away from the wearer's body (i.e. the outer surface 52 generally is formed by at least a portion of the backsheet 26 and other components joined to the backsheet 26). The first waist region 56 and the second waist region 58 extend, respectively, from the end edges 64 of the periphery 60 to the lateral centerline 66 of the diaper 20. The waist regions each comprise a central region 68 and a pair of side panels which typically comprise the outer lateral portions of the waist regions. The side panels positioned in the first waist region 56 are designated 70 while the side panels in the second waist region 58 are designated 72. While it is not necessary that the pairs of side panels or each side panel be identical, they are preferably mirror images one of the other. In a preferred embodiment of the present invention, the side panels 72 positioned in the second waist region 58 are elastically extensible in the lateral direction (i.e. elasticized side panels 30). (The lateral direction (x direction or width) is defined as the direction parallel to the lateral centerline 66 of the diaper 20; the longitudinal direction (y direction or length) being defined as the direction parallel to the longitudinal centerline 67; and the axial direction (Z direction or thickness) being defined as the direction extending through the thickness of the diaper 20).

Figure 1 shows a preferred embodiment of the diaper 20 in which the topsheet 24 and the backsheet 26 have length and width dimensions generally larger than those of the absorbent core 28. The topsheet 24 and the backsheet 26 extend beyond the edges of the absorbent core 28 to thereby form the periphery 60 of the diaper 20. The periphery 60 defines the outer perimeter or, in other words, the edges of the diaper 20. The periphery 60 comprises the longitudinal edges 62 and the end edges 64.

The containment assembly 22 of the diaper 20 is shown in Figure 1 as comprising the main body (chassis) of the diaper 20. The containment assembly 22 comprises at least an absorbent core 28 and preferably an outer covering layer comprising the topsheet 24 and the backsheet 26. When the absorbent article comprises a separate holder and a liner, the containment assembly 22 generally comprises the holder and the liner (i.e. the containment assembly 22 comprises one or more layers of material to define the holder while the liner comprises an absorbent composite such as a topsheet, a backsheet, and an absorbent core). For unitary absorbent articles, the containment assembly 22 comprises the main structure of the diaper with other features added to form the composite diaper structure. Thus, the containment assembly 22 for the diaper 20 generally comprises the topsheet 24, the backsheet 26, and the absorbent core 28.

A preferred embodiment of the diaper 20 has an asymmetric, modified T-shaped, absorbent core 28 having ears 102 in the first waist region 56 but a generally rectangular shape in the second waist region 58. This configuration allows wider elasticized side panels 30 in the second waist region 58. An exemplary absorbent structure for use as the absorbent core 28 of the present invention that has achieved wide acceptance and commercial success is described in US Patent 4,610,678 entitled "high-Density Absorbent Structures" issued to Weisman and Goldman on September 9, 1986. US Patent 4,673,402 entitled "Absorbent Articles With Dual-Layered Cores" issued to Weisman, Houghton, and Gellert on June 16, 1987; and US Patent 4,888,231 entitled "Absorbent Core Having A Dusting Layer" issued to Angstadt on December 19, 1989; also describe absorbent structures that are useful in the present invention. The absorbent core 28 is preferably the commercially successful absorbent member described in US Patent 4,834,735, entitled "High Density Absorbent Members Having Lower Density and Lower Basis Weight Acquisition Zones", issued to Alemany and Berg on May 30, 1989. Each of these references are incorporated herein by reference. The diaper 20 preferably further comprises elasticized leg cuffs 32 for providing improved containment of liquids and other body exudates. Each elasticized leg cuff 32 may comprise several different embodiments for reducing the leakage of body exudates in the leg regions. Each of these patents are incorporated herein by reference. While each elasticized leg cuff 32 may be configured so as to be similar to any of the leg bands, side flaps, barrier cuffs, or elastic cuffs described above, it is preferred that each elasticized leg cuff 32 comprise at least an inner barrier cuff 84 comprising a barrier flap 85 and a spacing elastic member 86 such as described in the above-referenced US Patent 4,909,803. In a preferred embodiment, the elasticized leg cuff 32 additionally comprises an elastic gasketing cuff 204 with one or more elastic strands 105, positioned outboard of the barrier cuff 84 such as described in the above-references US Patent 4,695,278.

The diaper 20 may further comprise an elastic waist feature 34 that provides improved fit and containment. The elastic waist feature 34 at least extends longitudinally outwardly from at least one of the waist edges 83 of the absorbent core 28 in at least the central region 68 and generally forms at least a portion of the end edge 64 of the diaper 20. Thus, the elastic waist feature 34 comprises that portion of the diaper at least extending from the waist edge 83 of the absorbent core 28 to the end edge 64 of the diaper 20 and is intended to be placed adjacent the wearer's waist. Disposable diapers are generally constructed so as to have two elastic waist features, one positioned in the first waist region and one positioned in the second waist region. While a disposable diaper of the present invention can be constructed with a single elastic waist feature encircling the wearer, the discussion regarding the elastic waist feature will focus on diapers having a pair of elastic waist features, at least one, and preferably both, being constructed according to the present invention. Further, while the elastic waist feature or any of its constituent elements can comprise a separate element affixed to the containment assembly 22 of the diaper 20, the elastic waist feature 34 will be described with respect to a preferred embodiment in which the elastic waist feature 34 is constructed as an extension of other elements of the diaper such as the backsheet 26 or the topsheet 24, preferably both the backsheet 26 and the topsheet 24.

A more detailed description of the components of the diaper is given in WO 93/16669 which is incorporated herein by reference.

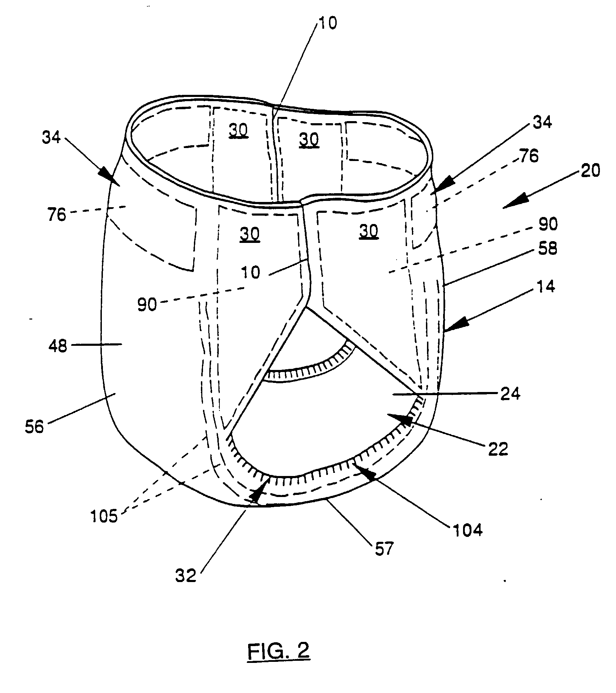

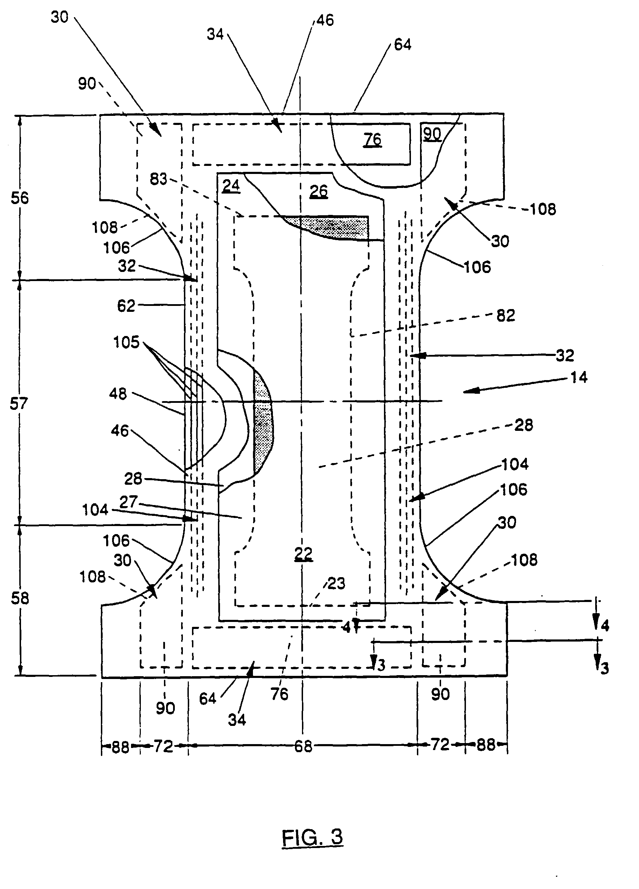

Figures 2 and 3 show a training pant which is a further example of an absorbent article according to the invention. The training pants 20 comprise a chassis 14, side seems 10, and an absorbent assembly 22. The chassis 14 will have at least a front portion 56, a rear portion 58, a crotch portion 57, longitudinal side regions 88, and ear flaps 72 and will comprise an elastic ear flap member 90 operatively associated with each ear flap 72 to form a laminated ear flap which will be elastically activated by a mechanical stretching process which will be described in greater detail herein below. The absorbent assembly 22 is secured to the chassis 14.

The outer layer 48 is that portion of the chassis 14 which will form the exterior of the disposable training pants 20, i.e. face away from the wearer. The outer layer 48 is compliant, soft feeling, and non-irritating to the wearer's skin.

The inner layer 46 is that portion of the chassis 14 which will form the interior of the chassis 14, and will contact at least the waist and legs of the wearer. The inner layer is also compliant, soft feeling, and on-irritating to the wearer's skin.

The inner layer 46 is preferably positioned adjacent to the outer layer 48 and is preferably Joined thereto by attachment means (not shown) such as those well known in the art. For example, the inner layer 46 may be secured to the outer layer 48 by a uniform continuous layer of adhesive, a patterned layer of adhesive, or an array of separate lines, spirals, or spots of adhesive.

In a preferred embodiment, at least a portion of the chassis inner and outer layers 46, 48 will be subjected to mechanical stretching in order to provide a "zero strain" stretch laminate that forms the elasticized ear flaps 30. Thus, the inner and outer layers 46, 48 are preferably elongatable, most preferably drawable, but not necessarily elastomeric, so that the inner and outer layers 46, 48 will, upon mechanical stretching, be at least to a degree permanently elongated such that they will not fully return to their original undistorted configuration. In preferred embodiments, the inner and outer layers 46, 48 can be subjected to mechanical stretching without undue rupturing or tearing. Thus, it is preferred that the inner and outer layers 46, have a low cross-machine direction (lateral direction) yield strength.

The chassis 14 of the disposable training pants 20 preferably further comprises elasticized leg cuffs 32 for providing improved containment of liquids and other body exudates. Each elasticized leg cuff 32 may comprise several different embodiments for reducing the leakage of body exudates in the leg regions. While each elasticized leg cuff 32 may be configured so as to be similar to any of the leg bands, side flaps, barrier cuffs, or elastic cuffs described above, it is preferred that each elasticized leg cuff 32 comprise at least a side flap 104 and one or more elastic strands 105.

The chassis 14 of the disposable training pants 20 further preferably comprises an elasticized waistband 34 disposed adjacent the end edge 64 of the disposable training pants 20 in at least the rear portion 58, and more preferably has an elasticized waistband 34 disposed in both the front portion 56 and the rear portion 58. The waistband of the disposable training pants 20 is that portion which is intended to be placed adjacent the wearer's waist. The elasticized waistband 34 provides a member that maintains a defined area coverage, contacts the wearer's waist, and is elastically extensible in at least the lateral direction so as to dynamically fit against the waist of the wearer and to dynamically conform to the waist of the wearer so as to provide improved fit. Thus, the waistband is generally that portion of the disposable training pants 20 extending from the end edge 64 of the disposable training pants 20 to at least the waist edge 83 of the absorbent core 28. While the elasticized waistband 34 can comprise a separate element affixed to the chassis 14 of the disposable training pants 20, the waistband is preferably an extension of other elements of the disposable training pants 20 such as the inner layer 46, the outer layer 48, or any combination of these elements and an elastomeric material joined thereto. Alternatively, the topsheet 24 and the backsheet 26 of the absorbent assembly 22, may extend beyond the edges of the absorbent core 28 and have an elastomeric material Joined thereto to form an elasticized waistband. Disposable training-pants are often constructed so as to have two elasticized waistbands; one positioned in the front portion 56 and one positioned in the rear portion 58. The disposable training pants 20 at least has an elasticized waistband 34 disposed in at least the central region 68 of the rear portion 58. Preferably another elasticized waistband is disposed on the front portion 56. Preferably both elasticized waistbands 34 are disposed between the elasticized ear flaps 30.

In a preferred embodiment, the chassis 14 comprises elasticized ear flaps 30 in the front portion 56 and the rear portion 58. The elasticized ear flaps 30 are unitary elements of the chassis, i.e. they are not separately manipulative elements secured to the chassis, but rather are formed from and are extensions of the chassis materials. The elasticized ear flaps 30 provide an elastically extensible feature that provides a more comfortable and contouring fit by initially conformably fitting the disposable garment to the wearer and sustaining this fit throughout the time of wear well past when the disposable garment has been loaded with exudates since the elasticized ear flaps allow the sides of the disposable garment to expand and contract.

Each ear flap 72 comprises that portion of the chassis 14 that extends laterally outwardly from and along the central region 68 of the chassis 14 to the longitudinal side region 88 of the chassis 14. The ear flap 72 generally extends longitudinally from the end edge 64 of the chassis 14 to the portions of the longitudinal edge 62 of the chassis 14 that forms the leg opening (this segment of the longitudinal edge 62 being designated as leg edge 106). In a preferred embodiment of the present invention, each ear flap is formed by the portions of the inner layer 46 and the outer layer 48 that extend beyond the central region 68 of the chassis 14.

In an embodiment of the present invention, the elastic ear flap members 90 are operatively associated with the chassis 14 in the ear flaps 72, preferably between the inner layer 46 and the outer layer 48, so that the elastic ear flap members 90 allow the elasticized ear flaps 30 to be elastically extensible in the lateral direction (laterally elastically extensible). As used herein, the term "elastically extensible" means a segment or portion of the chassis that will elongate in at least one direction (preferably the lateral direction for the ear flaps and the waistbands) when tensional forces (typically lateral tensional forces for the ear flaps and the waistbands) are applied, and will return to about its previous size and configuration when the tensional forces are removed. Generally, elastomeric materials useful in the present invention will contractively return to at least about 75% of their original configuration within about 5 seconds or less upon stretch and immediate release thereof (i.e. a "snappy" elastic).

Generally, therefore, the absorbent core of a absorbent article according to the invention may be any absorbent means which is generally compressible, conformable, non-irritating to the wearer's skin, and capable of absorbing and retaining liquids such as urine and other body exudates. The absorbent core has a garment surface, a body surface, side edges, and waist edges.

The absorbent core may be manufactured in a wide variety of sizes and shapes (e.g., rectangular, hourglass, "T"-shaped, asymmetric, etc.) and from a wide variety of liquid-absorbent materials commonly used in disposable diapers and other absorbent articles such as comminuted wood pulp which is generally referred to as airfelt. Examples of other suitable absorbent materials include creped cellulose wadding, meltblown polymers including coform, chemically modified or cross-linked cellulosic fibers, tissue including tissue wraps and tissue laminates, absorbent foams, absorbent sponges, superabsorbent polymers, absorbent gelling material, or any equivalent material or combinations of material. The configuration and construction of the absorbent core may also be varied (e.g., the absorbent core may have varying caliper zones, a hydrophilic gradient, a superabsorbent gradient, or lower average density and lower average basis weight acquisition zones; or may comprise one or more layers or structures). The total absorbent capacity of the absorbent core should, however, be compatible with the design loading and the intended use of the diaper. Further, the size and absorbent capacity of the absorbent core may be varied to accommodate wearers ranging from infants through to adults.

A preferred embodiment of a diaper has an asymmetric, modified T-shaped, absorbent core having ears in the first waist region but a generally rectangular shape in the second waist region. This configuration allows wider elasticized side panels in the second waist region. An exemplary absorbent structure for use as the absorbent core of the present invention that has achieved wide acceptance and commercial success is described in U.S. Patent 4,610,678 entitled "High-Density Absorbent Structures" issued to Weisman and Goldman on September 9, 1986. U.S. Patent 4,673,402 entitled "Absorbent Articles With Dual-Layered Cores" issued to Weisman, Houghton, and Gellert on June 16, 1987; and U.S. Patent 4,888,231 entitled "Absorbent Core Having A Dusting Layer" issued to Angstadt on December 19, 1989; also describe absorbent structures that are useful in the present invention. The absorbent core may also comprise the commercially successful absorbent member described in U.S. Patent 4,834,735, entitled "High Density Absorbent Members Having Lower Density and Lower Basis Weight Acquisition Zones", issued to Alemany and Berg on May 30, 1989. The absorbent core may further comprise the dual core system containing an acquisition/distribution core of chemically stiffened fibers positioned over the absorbent storage cores as detailed above such as that system described in co-pending U.S. Patent Application Serial No. 07/843,706, "Absorbent Article with Elastic Waist Feature and Enhanced Absorbency", Clear and Alemany, filed on February 28, 1992. Each of these reference are incorporated herein by reference.

The backsheet is positioned adjacent the garment surface of the absorbent core and is preferably joined thereto by attachment means such as those well known in the art. For example, the backsheet may be secured to the absorbent core by a uniform continuous layer of adhesive, a patterned layer of adhesive, or an array of separate lines, spirals, or spots of adhesive. Adhesives which have been found to be satisfactory are manufactured by Century Adhesives, Inc. of Columbus, Ohio and marketed as Century 5227; and by H. B. Fuller Company of St. Paul, Minnesota and marketed as HL-1258. The attachment means will preferably comprise an open pattern network of filaments of adhesive as is disclosed in U.S. Patent 4,573,986 entitled "Disposable Waste-Containment Garment", which issued to Minetola and Tucker on March 4, 1986, and which is incorporated herein by reference. An exemplary attachment means of an open pattern network of filaments comprises several lines of adhesive filaments swirled into a spiral pattern such as is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Each of these patents are incorporated herein by reference. Alternatively, the attachment means may comprise heat bonds, pressure bonds, ultrasonic bonds, dynamic mechanical bonds, or any other suitable attachment means or combinations of these attachment means as are known in the art.

The backsheet is impervious to liquids (e.g., urine) and is preferably manufactured from a thin plastic film, although other flexible liquid impervious materials may also be used. As used herein, the term "flexible" refers to materials which are compliant and will readily conform to the general shape and contours of the human body. The backsheet prevents the exudates absorbed and contained in the absorbent core from wetting articles which contact the diaper such as bedsheets and undergarments. The backsheet may thus comprise a woven or nonwoven material, polymeric films such as thermoplastic films of polyethylene or polypropylene, or composite materials such as a film-coated nonwoven material. Preferably, the backsheet is a thermoplastic film having a thickness of from about 0.012 mm (0.5 mil to about 0.051 mm (2.0 mils).

The size of the backsheet is dictated by the size of the absorbent core and the exact diaper design selected. In a preferred embodiment, the backsheet has a modified hourglass shape extending beyond the absorbent core a minimum distance of at least about 1.3 cm to about 2.5 cm (about 0.5 to about 1.0 inch) around the entire diaper periphery. Preferably, the backsheet is much wider than the absorbent core in the second waist region so that the side panels in the second waist region are generally wider in the lateral direction than the side panels in the first waist region.

The topsheet is positioned adjacent the body surface of the absorbent core and is preferably joined thereto and to the backsheet by attachment means such as those well known in the art. Suitable attachment means are described with respect to joining the backsheet to the absorbent core. As used herein, the term "joined" encompasses configurations whereby an element is directly secured to the other element by affixing the element directly to the other element, and configurations whereby the element is indirectly secured to the other element by affixing the element to intermediate member(s) which in turn is affixed to the other element. In a preferred embodiment of the invention, the topsheet and the backsheet are joined directly to each other in the diaper periphery and are indirectly joined together by directly joining them to the absorbent core by the attachment means (not shown).

The topsheet is compliant, soft feeling, and non-irritating to the wearer's skin. Furthermore, the topsheet is liquid pervious, permitting liquids, for example, urine to readily penetrate through its thickness. A suitable topsheet may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. Preferably, the topsheet is made of a hydrophobic material to isolate the wearer's skin from liquids contained in the absorbent core.

There are a number of manufacturing techniques which may be used to manufacture the topsheet. For example, the topsheet may be a nonwoven web of fibers. When the topsheet comprises a nonwoven web, the web may be spunbonded, carded, wet-laid, meltblown, hydroentangled, combinations of the above, or the like. A preferred topsheet is carded and thermally bonded by means well known to those skilled in the fabrics art. A preferred topsheet comprises staple length polypropylene fibers having a denier of about 2.2. As used herein, the term "staple length fibers" refers to those fibers having a length of at least about 15.9 mm (0.625 inches). Preferably, the topsheet has a basis weight from about 18 to about 25 grams per square meter. A suitable topsheet is manufacture by Veratec, Inc., a Division of International Paper Company, of Walpole, Massachusetts under the designation P-8.

A diaper according to the invention preferably further comprises an elastic leg feature, elasticized leg cuffs, for providing improved containment of liquids and other body exudates. Each elasticized leg cuff may comprise several different embodiments for reducing the leakage of body exudates in the leg regions. (The leg cuff can be and is sometimes also referred to as leg bands, side flaps, barrier cuffs, or elastic cuffs.) U.S. Patent 3,860,003 entitled "Contractable Side Portions For a Disposable Diaper" issued to Buell on January 14, 1975, describes a disposable diaper which provides a contractible leg opening having a side flap and one or more elastic members to provide an elasticized leg cuff (gasketing cuff) extending laterally outwardly from the side edge of the absorbent core. U.S. Patent 4,909,803 entitled "Disposable Absorbent Article Having Elasticized Flaps" issued to Aziz and Blaney on March 20, 1990, describes a disposable diaper having "stand-up" elasticized flaps (barrier cuffs) to improve the containment of the leg regions. U.S. Patent 4,695,278 entitled "Absorbent Article Having Dual Cuffs" issued to Lawson on September 22, 1987, describes a disposable diaper having dual cuffs including a gasketing cuff and a barrier cuff. U.S. Patent 4,707,115 entitled "Disposable Waist Containment Garment" issued to Buell on November 3, 1987, discloses a disposable diaper or incontinent garment having side-edge-leakage-guard gutters configured to contain free liquids within the garment. Each of these patents is incorporated herein by reference. In a preferred embodiment, the elasticized leg cuff additionally comprises an elastic gasketing cuff with one or more elastic strands, positioned outboard of the barrier cuff such as described in the above-referenced U.S. Patent 4,695,278.

The diaper may further comprise an elastic waist feature that provides improved fit and containment. The elastic waist feature at least extends longitudinally outwardly from at least one of the waist edges of the absorbent core in at least the central region and generally forms at least a portion of the end edge of the diaper. Thus, the elastic waist feature comprises that portion of the diaper at least extending from the waist edge of the absorbent core to the end edge of the diaper and is intended to be placed adjacent the wearer's waist. Disposable diapers are generally constructed so as to have two elastic waist features, one positioned in the first waist region and one positioned in the second waist region. The elastic waist feature or any of its constituent elements can comprise a separate element affixed to the containment assembly of the diaper. Alternatively, the elastic waist feature can be constructed as an extension of other elements of the diaper such as the backsheet or the topsheet preferably, both the backsheet and the topsheet.

Examples of other absorbent articles and the use of the elastic feature of the invention therein, are the diaper with side panel described in WO 93/16669; an improved shaped diaper with side panel described in WO 94/28842; a training pant described in WO 95/00096; a baby diaper having an emetically rectangular body with side panels attached to it described in WO 95/3775. All the examples are incorporated herein by reference.

Elastomeric materials useful in the invention are available in the prior art and include, in particular, those materials taught by European patent no. 0 217 032, which is incorporated herein by reference.

A suitable material for making, for example, melt blown elastomeric fibers are block copolymers having the general formula A-B-A' where A and A' are each a thermoplastic polymer endblock which contains a styrenic moiety such as a poly(vinyl arene) and where B is an elastomeric polymer midblock such as conjugated diene or a lower alkene polymer. Suitable elastomeric materials include, but are not limited to, materials for forming elastomeric fibrous nonwoven webs and are ones in which the A and A' endblocks are selected from the group including polystyrene and polystyrene homologs such as poly(alpha methylstyrene) and the B midblock is either polybutadiene, polyisoprene or poly (ethylene-butylene) (examples are disclosed in U.S. patent no. 4,333,782, 4,323,534 and 4,355,425 incorporated herein).

U.S. patent no. 4,418,123, which is incorporated herein by reference, describes A-B-A block copolymers having styrenic endblocks A and amorphous intermediate blocks B. Commercially available A-B-A' block copolymers having a saturated or essentially saturated poly(ethylene-butylene) mid block or segment B are sometimes referred to as polystyrene/poly(ethylene-butylene)/polystyrene block copolymers (S-EB-S), and are available under the trademark KRATON G, for example, KRATON G 1650, KRATON G 1652 and KRATON GX 1657 from Shell Chemical Company. KRATON rubber materials are described in detail in a number of Shell Chemical Company publications including, SC: 198-83, 7/83 5M. Other materials available from Shell Chemical Company, sometimes referred to as S-B-S block copolymers, are designated KRATON D, for example, KRATON D 1101, KRATON D 1102 and KRATON D 1116.

The A-B-A' block copolymers may be extruded to produce elastomeric materials, particularly elastomeric films and elastomeric fibers, more particularly, elastomeric microfibers by meltblowing. The S-EB-S thermoplastic block copolymer provides a material which provides satisfactory elastic and strength properties even when containing a rather high content of polyolefin material.

Another S-B-S block copolymer material is commercially available under the trade designation Solprene 418 from the Phillips Petroleum Company.

Other elastomeric resins which may be used are A-B-A' block copolymers referred to as S-I-S block copolymers and are available from the Shell Chemical Company under the trade designation KRATON D, for example, KRATON D 1107, KRATON D 1111, KRATON D 1112 and KRATON D 1117.

Other exemplary elastomeric materials for use in formation of films include polyester elastomeric materials such as, for example, polyurethane elastomeric materials such as, for example, those available under the trademark ESTANE from B. F. Goodrich & Co. and polyamide elastomeric materials such as, for example, those available under the trademark PEBAX from the Rilsan Company. Generally, any suitable elastomeric fiber forming resins or blends containing the same may be utilized for the nonwoven webs of elastomeric fibers of the invention and any suitable elastomeric film forming resins or blends containing the same may be utilized for the elastomeric films of the invention.

The elastomeric film forming resin used in the invention may essentially consist of an elastomeric S-EB-S thermoplastic resin which typically may contain plasticizers, pigments, antioxidants and other conventionally employed additives. Further, the S-EB-S block copolymers may be blended with polyolefins, e.g., polyethylene and/or polypropylene. The polyolefin which is utilized in blending the S-EB-S block copolymers must be one which, when blended with the S-EB-S block copolymer and subjected to an appropriate combination of elevated pressure and elevated temperature conditions is extrudable in blended form with the block copolymer. In particular, preferred polyolefin materials include polyethylene, polypropylene and polybutene, including ethylene copolymers, propylene copolymers and butene copolymers. Blends of two or more of the polyolefins may be utilized. A particularly preferred polyethylene may be obtained from U.S.I. Chemical Company under the trade designation Petrothene Na601. (Also referred to as PE Na601 or Na601.) A suitable polypropylene may be obtained from the Himont Corporation under the trade designation PC-973.

Any conventionally known polymer as the non-elastomeric material may be useful in the invention. Examples include, but are not limited to commercially available polyesters, polyamides (PE, PP), polyolefins etc., PP and PE films are particularly suitable and, in particular, PE having a density of more than 0.917 g/cm³ is preferred. Preferably, the properties of the non-elastomeric material are low force and low elongation at break, for example, for freeing of the elastic properties in, for example, the CD-direction.

Examples of elastomeric materials and non-elastomeric materials which can be extruded or co-extruded to form a film include any combination of the known elastomeric materials and non-elastomeric materials described hereinbefore. In particular, blends of kratons and PE or PP are suitable.

The elastic feature comprises at least one layer formed from an elastomeric material and at least one layer formed from a substantially non elastomeric material, the two layers being extruded to form a film. Extrusion can be any conventionally known method, for example, reference is made to the method described in US Patent No 5,147,346 (Clopay), the details of which are incorporated herein by reference.

Coextrusion techniques are well known to those versed in the art and need not be discussed in detail. One such technique for coextruding films includes the use of a feed block located between separate extruders and a die. The feed block serves to combine various thermoplastic resin streams flowing from the separate extruders into intimate contact with one another to form a single stream and to direct the combined streams in a parallel flow pattern into the die for formation of the coextruded composite film. In the even that varying widths are desired with respect to each layer of the composite film and especially the outer layer, converging dies, each having a different width, can be employed to combine the thermoplastic streams flowing out from the separate extruders into intimate and overlapping contact with one another. With this method, however, the feed block is eliminated, and for each extruder employed, a corresponding die is utilized for receiving the thermoplastic stream flowing out therefrom. It should of course be understood that when the composite film is coextruded with the outer layer having a narrower width, the die corresponding to the outer layer will have a width which is less than that of the die corresponding to the inner layer. See Example IV discussed hereinafter.

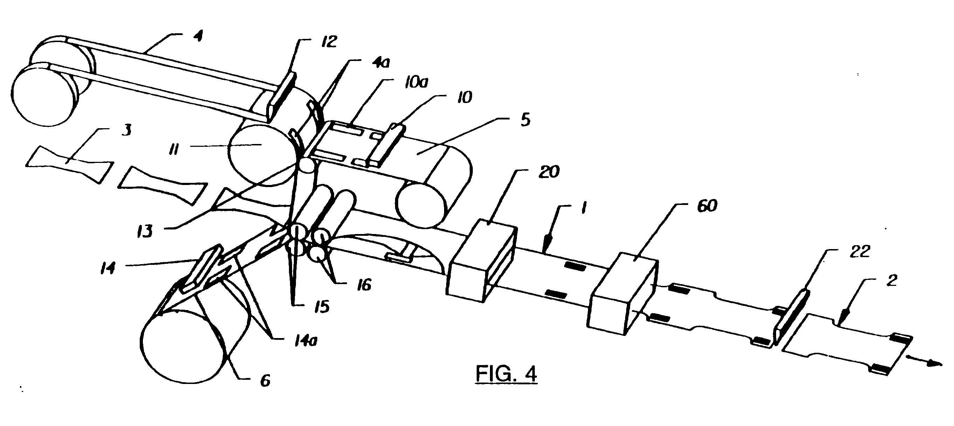

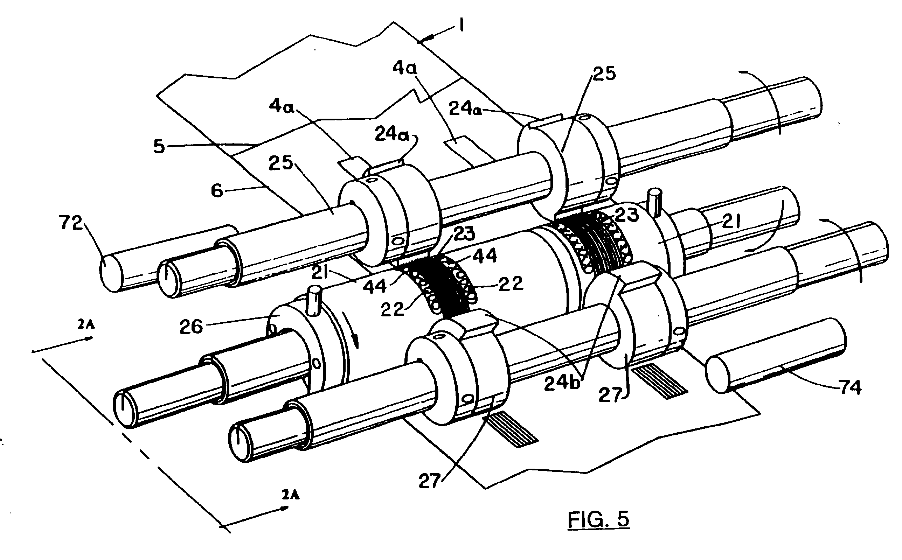

Figure 4 illustrates one example of the manufacture of absorbent articles according to the invention.

There is shown a continuous web 1 comprised of a plurality of interconnected single use diapers 2. Each diaper is comprised of an absorbent pad element 3, a pair of elastomeric elements or patches 4a, which may be comprised of "live" synthetic or natural rubber, synthetic or natural rubber foam, elastomeric film, elastomeric nonwoven laminate, elastomeric scrim or the like, secured to the webs at predetermined spaced locations, said absorbent pad. Preferably, the elastomeric patches comprise an extruded film according to the invention comprising at least one layer of elastomeric material and at least one layer of non-elastomeric material. Before activation, the elastomeric properties are restrained by the extruded non-elastomeric layer. Said elastomeric patches are located intermediate a moisture-impervious backsheet 5, which is typically comprised of an elongatable polymeric material such as one mil thick polyethylene film, and a moisture-pervious topsheet 6, which is typically comprised of either an elongate non-woven fibrous material or an elongatable apertured polymeric film.

Other materials which can be used for elastomeric elements or patches 4a which may additionally be used in the absorbent article comprise foams having an elongation to break of at least about 400% and an extension force of about 200 grams per inch of sample width at 50% extension of its unstrained length. Exemplary foams which have been found usable are: General Foam polyurethane foam No. 40310 having a no-load caliper or thickness of approximately 80 mils (2032µm) and a density of approximately 2.06 lbs/cu.ft (approximately 0.033 grams per cubic centimeter), as available from General Foam of Paramus, N.J.; Bridgestone SG polyurethane foam having a no-load caliper or thickness of approximately 80 mils and a density of about 2.06 lbs/cu.ft (0.033 grams per cubic centimeter), as available from Bridgestone of Yokohama, Japan; cross-linked natural rubber foam having a no-load caliper or thickness of approximately 50 mils and a density of about 13.3 lbs/cu.ft (0.214 grams per cubic centimeter), as available from Fulflex Inc. of Middleton, R.I.; and cross-linked natural rubber foam having a no-load caliper or thickness of approximately 50 mils and a density of about 13.3 lbs/cu.ft (0.24 grams per cubic centimeter), as available from Ludlow Composites Corporation of Fremont, Ohio.

It is preferred that the elastomeric patches comprise the extruded film of the invention, wherein the elastomeric layer(s) and non-elastomeric layer(s) are extruded from, for example, different die-heads or are co-extruded from the some die-head. Extrusion can take place simultaneously with the manufacture of the absorbent article or else, the extruded films can be preformed before being assembled as shown in Figure 4. If the elastomeric film is premanufactured, then, as shown in Figure 4, two rolls of the elastomeric film 4 are fed under very slight (essentially "zero-strain") tension at a speed which provides the desired length of elastomeric patch 4a per diaper onto an anvil roll equipped with vacuum hold down parts (not shown) at its periphery. Knife 12 makes one cut per diaper and the substantially untensioned elastomeric patches 4a travel with anvil roll 11 secured to its periphery by vacuum until they reach transfer point 13.

Particularly preferred materials for backsheet 5 include blends comprised of about 45-90% linear low density polyethylene and about 10-55% polypropylene. If used in unembossed form, the backsheet 5 typically exhibits a no-load caliper or thickness of approximately 1 mil. If desired, the backsheet may be embossed to a caliper of approximately 5.5 mils to enhance the web's handling and appearance characteristics. Exemplary backsheet materials which have been found to work are: RR8220 blend REDEM, as available from Tredegar Industries, Inc. of Terre Haute, Ind.; and RR5475 blend ULAB, as available from Tredegar Industries, Inc. of Terre Haute, Ind.

One particularly preferred material for moisture pervious topsheet 6 comprises a hydrophobic, nonwoven carded web having a basis weight in the range of about 18-20 grams per square yard and comprised of approximately 2.2 denier polypropylene fibers, as available from Veratec, Inc., a Division of International Paper Company of Walpole, Mass. under the designation P8.

A particularly desirable aesthetic appearance results in the "zero-strain" stretch laminate portions of the diaper web when the backsheet 5, the topsheet 6 or both are comprised of resilient three-dimensional polymeric webs of the type generally disclosed in commonly assigned US Patent No 4,342,314 issued to Radel and Thompson on Aug. 31, 1982 and hereby incorporated herein by reference.

The continuous webs of backsheet material 5 and topsheet material 6 are preferably maintained under very slight (essentially "zero-strain") tension in the machine direction to prevent wrinkling and to facilitate registration with the diaper assembly and converting operations until the completed diaper web is severed into discrete diapers 2 at knife 22.

The diaper web forming operation is illustrated only schematically in Figure 4. The absorbent pad segments 3 are fed into the nip between a pair of combining or laminating rolls 15 at regularly spaced, predetermined intervals. In a particularly preferred embodiment, the absorbent pad segments 3 are preferably comprised of airfelt confined within a cellulosic tissue envelope to provide pad integrity in use.

"Zero-strain" - stretch laminate webs may be produced utilizing either an intermittent bonding configuration or a substantially continuous bonding configuration. The intermittent bonding configuration is normally desirable in those situations where in the substantially inelastic webs in the laminate are relatively elongatable or drawable without rupture and where a high degree of z-direction bulking is desired in the finished laminate.

Conversely, a continuous bonding configuration has generally been found desirable where the degree of z-direction bulking is not of prime importance and one or more of the relatively inelastic webs in the laminate is difficult to elongate or draw without causing rupture. In the latter situation, a substantially continuous bonding configuration maintains all of the layers of the laminate in relatively close adherence to one another after the incremental stretching operation. Accordingly, even if one or more of the relatively inelastic webs is damaged to the point of rupture during the incremental stretching operation, the relatively close adherence of the damaged portions of the relatively inelastic web or webs to the elastomeric ply makes it difficult for the end user to perceive that any damage has occurred. Provided rupture of the relatively inelastic web or webs does not defeat the web's intended functionality, e.g., fluid-imperviousness, the damage which does occur to the relatively inelastic web or webs during the incremental stretching operation is not perceived as a negative in the end product.

Thus, an unexpected benefit which results from the use of a continuous bonding configuration in particularly preferred "zero-strain" stretch laminate webs of the present invention is that it permits the manufacturer of the elasticized article to select from a much wider range of relatively inelastic webs which may be successfully employed in laminates of the present invention. In essence, it permits the use of relatively inelastic webs which would not normally be considered drawable to any appreciable extent in "zero-strain" stretch laminate webs of the present invention.

As can be seen in the embodiment of Figure 4, the continuous web of moisture-impervious elongatable backsheet material is directed in close proximity to a glue applicator 10. The glue applicator 10 may be used to apply a substantially uniform and continuous application of adhesive 10a to the backsheet 5 in those predetermined areas where the substantially untensioned elastomeric patches 4a will be placed. In a particularly preferred embodiment of the latter type, the adhesive selected is stretchable and the glue applicator comprises a melt blown applicating system.

One such melt blown adhesive applicating system which Applicant has found particularly well suited for producing a substantially continuously bonded "zero-strain" stretch laminate web of the present invention is a melt blown spray applicator Model No. GM-50-2-1-GH, as available from J&M Laboratories of Gainesville, Ga. The latter system employs a nozzle having 20 orifices per lineal inch, as measured in the cross-machine direction, each orifice measuring approximately 0.020 inches in diameter. A Findley H-2176 Hot Melt Adhesive, as available from Findley Adhesives of Findley, Ohio is preferably heated to a temperature of approximately 340°F. and applied to a backsheet 5 at a rate of approximately 7.5-10 milligrams per square inch. Heated compressed air at a temperature of approximately 425°F. and a pressure of approximately 50 psig is issued through the secondary orifices in the adhesive nozzle to assist in uniformly distributing the adhesive fibrils during the laydown operation.

The intimate contact of the hot glue substantially shown as 10a in Figure 4 with the backsheet web 5 for the time which passes prior to incremental stretching of the resultant "zero-strain" stretch laminate portion of the diaper web provides softening of the backsheet. For some webs, such as conventional polyethylene backsheet material, this softening has been found beneficial in minimizing damage to the backsheet during the incremental web stretching process. This may be particularly important in situations where the web in question imparts some function, e.g., fluid-imperviousness, to the finished article being produced.

Alternatively, the components comprising the "zero-strain" portions of the diaper web may be intermittently or continuously bonded to one another using unheated adhesive, heat bonding, pressure bonding, ultrasonic bonding, etc. In such instances, thermal energy may, if desired, be applied to the backsheet web 5 by other means well known to those skilled in the art, e.g., radiant heaters (not shown), hot air blasts (not shown), etc., to achieve a similar result.

Elastomeric material 4 is extruded (alternatively co-extruded with the non-elastomeric material) at the desired place on backsheet 5 to form the extruded elastic composite of the invention, preferably as a co-extruded film. The material can be extruded directly onto the backsheet or can be preformed as an extruded film and adhered as shown. At point 13, the extruded film 4a is transferred to predetermined portions of the backsheet web 5 coiniciding with, for example, adhesive 10a, preferably by high pressure air blasts. The transfer is sequential and the surface speed of the vacuum equipped anvil roll and backsheet web 5 are essentially equal. Alternatively, to avoid the adhesive, preferably the elastomeric layer and non-elastomeric layer are extruded directly onto the backsheet at the predetermined points relating to the elastic feature of the absorbent article. Alternatively, one or more elastic feature may comprise an elastomeric patch conventionally known from the prior art, adhered as described hereinbefore with the adhesive patches 10a and furthermore, one or more elastic feature may comprise the elastomeric film of the invention.

The backsheet web 5 with elastomeric patches 4a attached thereto at predetermined points along its length is then directed to the pair of laminating or combining rolls 15.

A continuous web of a moisture-pervious topsheet material 6, such as an elongatable fibrous nonwoven web, is directed in close proximity to a second glue applicator 14 where a pattern of adhesive 14a sized to substantially match the dimensions and locations of the elastomeric patches 4a, preferably extrude elastomeric/non-elastomeric composite on backsheet web 5 is preferably applied. As with the backsheet material 5, the pattern of adhesive applied to the topsheet material 6 may be either intermittent or substantially continuous, depending on the properties of the topsheet material 6 and the characteristics desired in the resultant "zero-strain" stretch laminate web. If desired, adhesive applicator 14 may be identical to adhesive applicator 10.

The backsheet web 5 and topsheet web 6 and the absorbent pads 3 are brought into contact with one another at combining rolls 15. Just prior to the webs and pads coming into contact with one another, additional adhesive is preferably applied to one or both webs by means which are, for clarity, not shown in Figure 3. The latter adhesive secures predetermined portions of the backsheet, the topsheet and the absorbent pad to one another to form the diaper web 1.

The fully assembled diaper web thereafter preferably proceeds through a pair of bond setting rolls 16, which may require chilling to minimize glue bleed through.

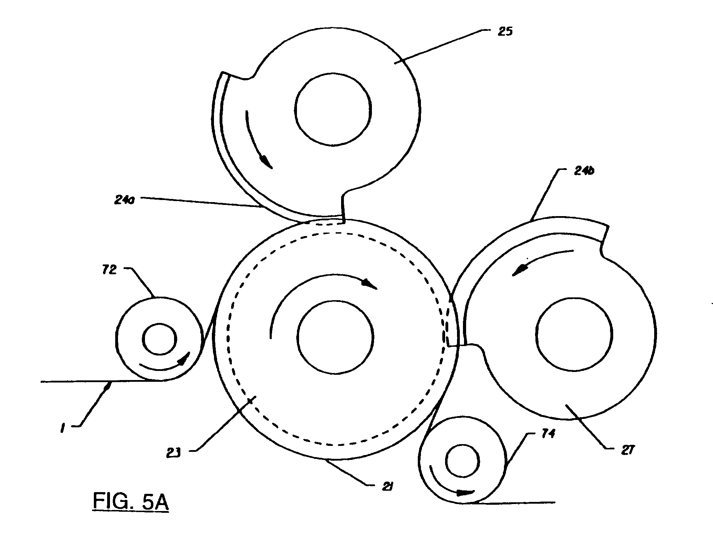

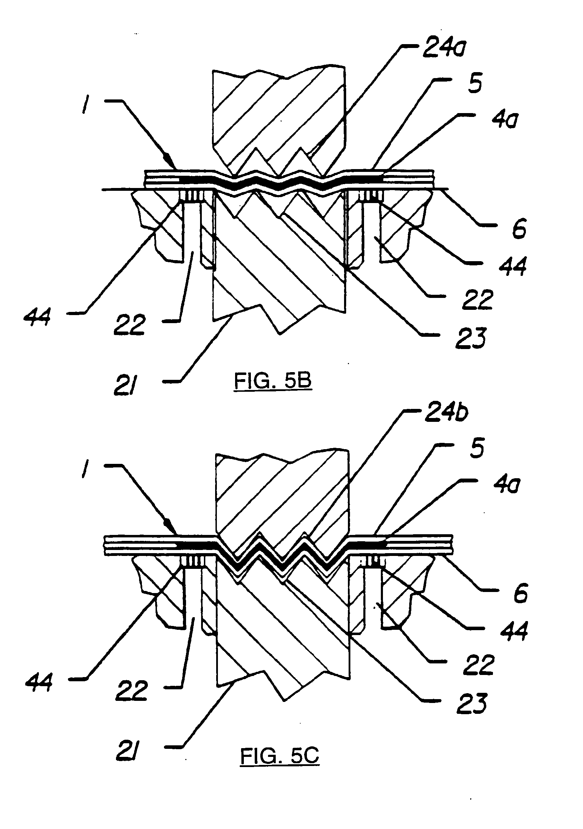

The fully assembled diaper web 1 is then directed through an incremental web stretching employing opposed pressure applicators having three dimensional surfaces which at least to a degree are complementary to one another system of the present invention, which is shown only schematically as 20 in Figure 4. Details of a particularly preferred incremental web stretching system of the present invention which can be employed as system 20 are set forth in Figure 5.

The elastomeric layer and substantially non-elastomeric layer form a film which is elasticized in defined areas either before, during or after incorporation into the final absorbent article, by any form of activation as described hereinbefore, and preferably by mechanical stretching to result in at least partial destruction due to breaking of the non-elastomeric layer of the film at discrete points. This results in the release of the elastic properties of the elastomeric layer of the film which were previously restrained when combined with the non-elastomeric layer to form a film. Mechanical stretching can be carried out by any method known in the prior art which, for example, includes tentering as described in US Patent No 5,244,482 CD-direction stretching), diffential-speed stretching (MD-direction stretching) etc..

Particularly preferred methods and apparatus used for mechanically stretching the extruded film use meshing corrugated rolls to mechanically stretch the film. A discussion of suitable apparatus and methods for mechanically stretching portions of a diaper is contained in U.S. Patent 4,107,364 issued to Sisson on August 15, 1978 and U.S. Patent 4,834,741 issued to Sabee on May 30, 1989. Particularly preferred apparatus and methods are disclosed in co-pending, commonly assigned, U.S. Patent Application Serial No. 07/662,536 entitled "Improved Method and Apparatus For Incrementally Stretching A Zero Strain Stretch Laminate Web To Impart Elasticity Thereto"; P&G Case 4339; allowed, filed by Gerald M. Weber et al. on February 28, 1991; U.S. Patent Application Serial No. 07/662,537 entitled "Improved Method And Apparatus For Incrementally Stretching Zero Strain Stretch Laminate Web In A Non-Uniform Manner To Impart A Varying Degree of Elasticity Thereto"; Procter & Gamble Case 4340; allowed, filed by Kenneth B. Buell et al. on February 28, 1991; and U.S. Patent Application Serial No. 07/662,543 entitled "Improved Method And Apparatus For Sequentially Stretching Zero Strain Stretch Laminate Web To Impart Elasticity Thereto Without Rupturing The Web"; Procter & Gamble Case 4341; allowed, filed by Gerald M. Weber et al. on February 28, 1991; the specifications and drawings of each one incorporated herein by reference.

A particularly preferred incremental stretching system which can be employed is shown in Figures 5; timing of the diaper web 1 containing substantially untensioned elastomeric patches 4a corresponding to the composite elastic feature of the invention comprising at least one layer of elastomeric material extruded with at least one layer of non-elastomeric material, is such that the substantially untensioned extruded film forming the elastomeric patches 4a contained within the diaper web substantially coincide with the corrugated or groove segments 24 contained on uppermost corrugated rolls 25 as the diaper web 1 passes between the segments 24 of uppermost corrugated rolls 25 and the continuously corrugated or grooved lowermost corrugated rolls 21. If desired, the grooved segments 24 may be of greater overall length than the elastomeric patches 4a, as measured in the machine direction, so as to impart a degree of extensibility to those portions of the topsheet and backsheet which are adjacent the elastomeric patches 4a in the finished diaper.

While the exact configuration, spacing and depth of the complementary grooves on the uppermost and lowermost corrugated rolls will vary, depending upon such factors as the amount of elasticity desired in the "zero-strain" stretch laminate portion of the fully processed web, two pairs of sequenced corrugated rolls, each having a peak-to-peak groove pitch of approximately 0.150 inches, an included angle of approximately 12° as measured at the peak, and a peak-to-valley groove depth of approximately 0.300 inches have been employed in a particularly preferred embodiment of the present invention. The exterior peak of each corrugation on the aforementioned corrugated roll pairs exhibits its a radius of approximately 0.010 inches, while the internal groove formed between adjacent corrugations typically exhibits a radius of approximately 0.040 inches. The sequenced corrugated roll pairs are typically adjusted so that the opposing peaks on each succeeding pair of meshing corrugated rolls increase their degree of overlap with one another approximately 0.035-0.050 inches from the first pair of meshing corrugated rolls to the second pair, the second set of meshing corrugated rolls typically overlapping one another to a total depth between bout 0.150 inches and about 0.175 inches.

The degree of overlap of the opposing peaks on the successive pairs of meshing corrugated rolls may of course be adjusted, as desired, to produce more or less extensibility in the resultant "zero-strain" stretch laminated portion of the web. For the aforementioned roll geometry and laminated web construction, peak-to-peak overlap depths ranging from as little as about 0.050 inches to as much as about 0.225 inches are feasible. In general, as the number of sequenced meshing corrugated roll pairs increases for any given total amount of incremental stretching, the potential for web damage decreases. This is believed due to the fact that the incremental stretching operation is carried out more gradually in a series of relatively small steps when a series of sequenced meshing corrugated roll pairs is employed. More gradual stretching of the web is believed to minimize damage to the web.

As can be seen from Figure 5, the diaper web 1 is caused by idler rolls 72, 74 to wrap the lowermost corrugated rolls 21 sufficiently to cover the active vacuum ports 22 located immediately adjacent each continuous set of grooves 23 on lowermost rolls 21. The vacuum ports 22, which are positioned so as to substantially coincide with the grooved segments 24 on uppermost corrugated rolls 25, are internally connected through rolls 21 to a pair of vacuum manifolds 26 which exert suction against the diaper web 1 as the diaper web is acted upon by the grooved segments 24 of uppermost corrugated rolls 25.

To minimize build up of either the adhesive, if used, to secured the untensioned elastomeric patches 4a to the fluid-pervious topsheet web 6 and/or patches 4a which are pre-formed extruded films according the invention or films extruded directly onto predetermined positions on the topsheet and/or backsheet, and the fluid-impervious backsheet web 5 or the adhesive used to secure the coinciding portions of the topsheet web and the backsheet web to one another, the grooved segments 24 on uppermost rolls 25 and the continuous grooves 23 on lowermost rolls 21 are preferably either comprised of a low friction materials, such as TEFLON ® (Polytetrafluoroethylene), or coated with a self-lubricating low friction material such as an aqueous dispersion of a fortified fluoropolymer, for example PERMALON® No 503 spray coating, as available from Micro Surface Corporation of Moris, Ill. Alternatively, the elastomeric layer and non-elastomeric layer is extruded or co-extruded directly onto the backsheet and/or topsheet of the absorbent article during the manufacturing process.

The vacuum ports 22 on lowermost rolls 21 are preferably covered by a porous material, such as 0.090" mesh honeycomb 44, to provide support to the portions of the diaper web 1 acted upon by the vacuum and to provide a good gripping surface against the web so as to substantially prevent lateral slippage or movement of the web across the honeycomb surface whenever the web is acted upon by vacuum.

Under optimum circumstances, the maximum degree of incremental stretching which can be imparted to the "zero-strain" portions of the diaper web 1 containing extruded elastomeric film 4a is determined by the depth of engagement between the grooves on segments 24 of uppermost corrugated rolls 25 and the continuous grooves 23 on lowermost corrugated rolls 21. However, unless the stretch laminate web is substantially prevented from slipping or contracting in a direction substantially parallel to the direction of web stretching as it passes between the meshing corrugated rolls, the optimum degree of incremental stretching is not realized. Therefore, in its most preferred form, the incremental web stretching operation is carried out while the outermost portions of all of three layers comprising the "zero-strain" stretch laminate composite are subjected to restrain, as generally shown in the cross-section of Figure 5B, to substantially prevent the "zero-strain" - stretch laminate portions of the diaper web from slipping or contracting in a direction parallel to the desired direction of stretching as it passes between the sets of sequentially positioned meshing corrugated rolls.

In another embodiment even rupture of one or more of the elongatable nonelastic webs may not render the resultant "zero-strain" - stretch laminate web unacceptable for its intended purpose, e.g., rupture of a backsheet web 5 does not necessarily destroy the laminate web's functionality for its intended purpose as long as one of the other plies in the laminate web provides the desired function in the finished article. For example, some degree of rupturing in the elongatable backsheet web 5 will not destroy the fluid-imperviousness of the resultant disposable diaper web if the composite elastic features corresponding to the elastomeric patches 4a are comprised of a fluid-impervious material. This is particularly true with respect to those "zero-strain" stretch laminate web embodiments employing substantially continuous bonding between the plies in question, since relatively close adherence of the plies to one another after incremental stretching renders such ply damage difficult to detect by the end user of the article.

Because the diaper web 1 shown in Figures 4-5 is substantially impervious to the passage of air by virtue of the presence of the uppermost moisture-impervious backsheet web 5, vacuum ports 22 covered by porous honeycomb material 44 can, if desired, be employed immediately adjacent each set of machine direction oriented grooves 23 in lowermost corrugated rolls 21. If the elastomeric patches 4a are sufficiently pervious to the passage of air, the suction forces generated by the vacuum will pass through the fluid-pervious topsheet web 6 and the elastomeric patches so as to tightly grip the overlying portions of the backsheet 5. In this instance, all three layers comprising the "zero-strain" - stretch laminate portions of the diaper web will be restrained during the incremental stretching operation.

If the elastomeric patches 4a were not substantially pervious to the passage of air, it would be necessary to either (a) position the vacuum ports 22 and the overlying honeycomb material 44 just outside the opposed edges of the elastomeric patches 4a so that suction forces could be exerted on the fluid-impervious drawable backsheet web 5 through the fluid-pervious drawable topsheet web 6; or (b) restrain all three layers comprising the "zero-strain" stretch laminate portions of the diaper web by means of suitable clamping apparatus capable of acting upon the opposed surfaces of the diaper web such apparatus are disclosed in the aforementioned concurrently filed, commonly assigned US patent application of Gerald M Weber, William R Vinage, Jr., Douglas H Benson and David A Sabatelli entitled IMPROVED METHOD AND APPARATUS FOR INCREMENTALLY STRETCHING ZERO STRAIN STRETCH LAMINATE WEB TO IMPART ELASTICITY THERETO, Serial No. 662,536 filed Feb. 28, 1991, the disclosure of which is hereby incorporated herein by reference.

The suction forces applied to the diaper web 1 shown in Figures 4-5 by vacuum ports 22 acting through porous honeycomb material 44 substantially prevent those portions of the diaper web containing substantially untensioned elastomeric and non-elastomeric extruded films shown as patches 4a from slipping or contracting in a laterally inward direction as they pass between the meshing portions of the continuous grooves 23 on lowermost corrugated rolls 21 and the grooves segments 24a and 24b of the first and second sets of uppermost corrugated rolls 25 and 27, respectively.

This not only maximizes the effectiveness of the incremental stretching by forcing the elongatable topsheet and backsheet webs secured to the patches 4a to undergo the fullest possible degree of elongation during the stretching operation, but also substantially prevents disproportionately high straining of the topsheet and/or backsheet webs to which they are secured in the areas immediately adjacent the opposed peripheral edge portions of the elastomeric patches.

Sequentially stretching the "zero-strain" stretch laminate portions of the diaper web 1 in accordance with the present invention using multiple pairs of meshing corrugated rolls, each subsequent pair of corrugated rolls exhibiting an increased degree of meshing, reduces the rate at which the elongation process is carried out and hence the rate at which strain is experienced by the composite web as it passes therebetween. In addition, the temporary release of tension from the web as it passes between the successive roll pairs allows some degree of stress redistribution to occur in the web prior to the web's being incrementally stretched to a greater degree by each succeeding roll pair.

Therefore, the more meshing roll pairs which are used to achieve the desired degree of incremental stretching, the more gradual will be the stretching of the web as it passes between any given roll pair and the more opportunities there will be provided for stress redistribution to occur within the web. This not only minimizes the rate at which strain is experienced by the composite web, but maximizes the opportunities for stress redistribution between each incremental stretching operation. Consequently a sequential roll system of the present invention is less prone to cause damage to the webs being processed than if the entire stretching operation is carried out on a single roll pair exhibiting an equivalent degree of meshing.

The extruded film can undergo incremental stretching as shown in Figure 5 before incorporation into the absorbent article as one or more elastic features, by combining with the topsheet and/or backsheet. In this case, the additional activation prior to incorporation into the absorbent article allows a more gentle subsequent activation of the diaper web 1, for example, which may avoid perforations appearing in, for example, the backsheet.

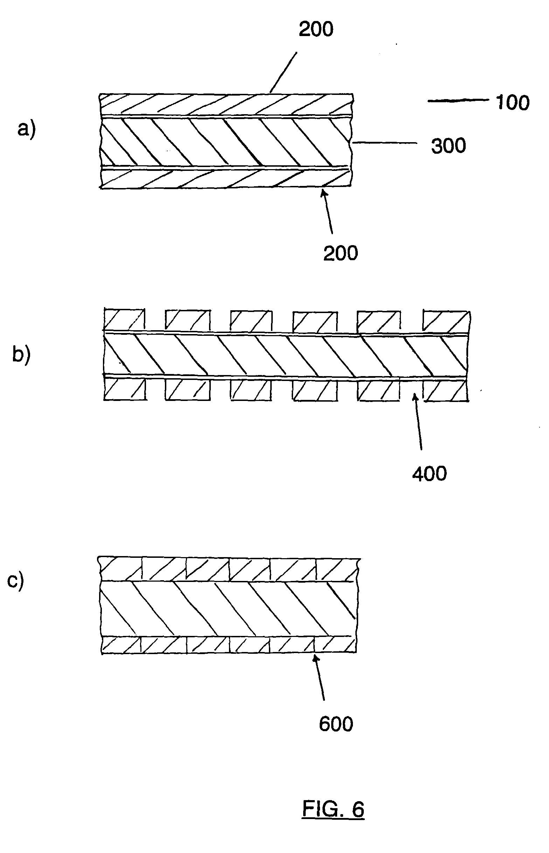

Figure 6 illustrates (a) a three-layer extruded laminate of the invention comprising two outer non-elastomeric layers 200 extruded to each form a film and sandwiched there between is an extruded elastomeric layer 300. Preferably, all three layers are co-extruded to form the laminate, before activation. The laminate depicted in (b) is according to the invention but after activation, for example, by incremental stretching as described with reference to Figures 5, 5A-5C. On activation, the properties of the non-elastomeric layers are preferably such, that they are brittle enough to break at predetermined points. Hence, the laminate of the invention at activation, for example, by stretching has interrupted outer non-elastomeric layers. As shown in (c), after activation and upon relaxation, the non-elastomeric layer is again in continuous contact 600 due to the elastic properties of the elastomeric layer. When activation is carried out using the method described in Figure 5, the locations of the ruptures in the non-elastomeric layer or layers corresponds to the position of the meshing rolls.

Following the sequential incremental stretching operation shown schematically as 20 in Figure 4, the fully assembled diaper web 1 is preferably passed through a side notching apparatus shown schematically as 60, wherein notches intended to coincide with the wearer's legs are cut from the lateral edge portions of the fully assembled diaper web.

Finally, the diaper web 1 is cut at predetermined locations along its length by means of knife 22 to produce hourglass-shaped single use diapers having at least one pair of substantially undamaged side panels which are elastically extensible, in a direction substantially parallel to the diaper's waistband, at least up to the point of initial stretching.

From the description contained herein, it is clear that the method and apparatus may be employed to advantage to produce a wide range of elasticized articles either comprised entirely of or including one or more discrete, isolated "zero-strain" stretch laminate web portions.

It is also recognized that while sequentially positioned pairs of meshing corrugated rolls having their corrugations aligned substantially parallel to one another are disclosed in the accompanying Figures, the present invention may be practised with equal facility employing sequentially positioned pairs of corrugated rolls wherein the corrugations are not all oriented parallel to one another. Furthermore, the corrugations on such sequentially positioned pairs of corrugated rolls need not necessarily be aligned parallel to either the machine or the cross-machine directions. For example, if a curvilinear waistband or legband portion is desired in a single use diaper constructed using the "zero-strain" stretch composite herein disclosed, the meshing teeth on the sequentially positioned pairs of corrugated rolls employed to sequentially stretch the "zero-strain" portions of the diaper web may be arrayed in the desired curvilinear configuration to produce elasticity along the desired curvilinear contour rather than in a straight line.

It is further recognized that while the preferred high speed processes herein disclosed employ sequentially positioned pairs of meshing cylindrical corrugated rolls, the sequential web stretching operation of the present invention may also be carried out utilizing a sequential intermittent stamping operation employing multiple sets of meshing platens, each set having a greater degree of meshing than the prior set, to sequentially stretch the "zero-strain" stretch laminate portions of a web or an article placed between the platens.

It is further recognized that while the preferred processes herein disclosed employ meshing cylindrical corrugated rolls, the web restraint principles may also be carried out utilizing an intermittent stamping operation employing meshing plates to incrementally stretch the web in question. In the latter instance, the only requirement is that the portions of the web to be incrementally stretched be adequately restrained by suitable vacuum or clamping means before the meshing plates are able to exert enough force on the web to cause slippage or contraction in a direction parallel to the direction of stretching.

The elastic feature of the absorbent article of the invention can have uni- or bi-directional or more stretch properties. The application of the elastic film in the absorbent article is not limited to any particular feature but can be placed at any position in the article to carry out or contribute to any feature of the article where the elastic properties are required. The elastic film can be located at more than one location in the article and can carry out more than one function - For example, the elastic film can have application as a stretch element in different regions of the absorbent article, with varying degrees of stretch or the same stretch properties and different directions of stretch or the same direction, from intermeshed regions, form CD/MD or other angular arrangements formed in one step (i.e. simultaneously) or in sequential steps.

According to a further embodiment, the composite elastic feature comprises an elastomeric film which has apertures, for example, said film is mechanically apertured by any available method, for example, by hot needling, vacuum forming, punching, slitting etc. Preferably, the apertures are formed by slitting or punching on-line during the manufacture of the absorbent article.

Although particular embodiments of the present invention have been illustrated and described herein, it would be obvious to those skilled in the art that various other changes and modifications can be made without departing from the scope of the invention. It is intended to cover in the appended claims all such changes and modifications that are within the scope of this invention.

## Claims

1. An absorbent article which comprises:
a liquid pervious topsheet,
a liquid impervious backsheet joined with said topsheet,
an absorbent core having side edges and waist edges, wherein said absorbent core is positioned between said topsheet and said backsheet, and
a composite elastic feature,
wherein said composite elastic feature comprises at least one layer formed from an elastomeric material and at least one layer formed from a substantially non-elastomeric material, wherein said layers are extruded to form a film, the film is elasticized by activation whereby the non-elastomeric material loses its restraining effect on the elastic properties of the elastomeric material.

2. An absorbent article according to claim 1 wherein an activation said non-elastomeric layer is caused to rupture at pre-determined points.

3. An absorbent article according to claim 1 wherein said non-elastomeric material is non-elongatable.

4. An absorbent article according to claim 1 wherein the elastomeric material and essentially non-elastomeric material are co-extruded to form a film.

5. An absorbent article according to any of claims 1 to 4 wherein activation is by mechanical stretching.

6. An absorbent article according to any of claims 1 to 5 wherein said film is elasticized by mechanically stretching said film to impart the elasticity thereto in the direction of stretching, at least up to the point of initial stretching.

7. An absorbent article according to any of claims 1 to 6 wherein said composite elastic feature forms at least a segment of said topsheet, a segment of said backsheet, or both.

8. AN absorbent article according to any of claims 1 to 7 wherein said composite elastic feature extends outwardly from at least one of the edges of the absorbent core.

9. An absorbent article according to any of claims 1 to 8 wherein said elastic feature comprises an elasticized leg cuff extending laterally outwardly from at least one of said side edges of said absorbent core.

10. An absorbent article according to any of claims 1 to 9 wherein said elastic feature comprises an elastic waist feature extending longitudinally outwardly from at least one of said waist edges of said absorbent core or overlaps at least one of said waist edges of said absorbent core.

11. An absorbent article according to any of claims 1 to 10 wherein said elastic feature comprises elasticized side panels, each of said elasticized side panels being elastically extensible in the lateral direction.

12. An absorbent article according to claim 7 wherein said composite elastic feature is integral with or co-extends with the backsheet.

13. An absorbent article according to claim 7 wherein said composite elastic feature is integral with or co-extends with the topsheet and said elastic feature further comprises a plurality of apertures.

14. An absorbent article according to any of claims 1 to 13 wherein activation occurs before the composite elastic feature is combined with any of the topsheet, backsheet or absorbent core.

15. An absorbent article according to any of claims 1 to 13 wherein activation takes place at the same time that the topsheet, backsheet and absorbent core are combined with each other and the elastic feature to assemble the absorbent article.

16. An absorbent article according to any of claims 1 to 13 wherein activation takes place after the topsheet, backsheet and absorbent core have been assembled with each other and with the elastic feature to form the absorbent article.

17. An absorbent article according to any of claims 1 to 16 wherein said composite elastic feature comprises apertures.

18. An absorbent article according to any of claims 1 to 17 wherein said composite elastic feature is apertured after the elastic feature has been combined with any of the topsheet, backsheet or absorbent core.
